# EUROPEAN PATENT SPECIFICATION

(11) **EP 2 716 326 B1**
(45) Date of publication and mention of the grant of the patent: **26.11.2014**
(21) Application number: 12187092.7
(22) Date of filing: 03.10.2012
(51) Int. Cl.: A61N 5/06

(54) **System for adjusting seasonal rhythm**
System zur Anpassung des jahreszeitlichen Rhythmus
Système de réglage de rythme saisonnier

(43) Date of publication of application: 09.04.2014
(73) Proprietor: Valkee Oy, 90590 Oulu (FI)
(72) Inventor: Nissilä, Juuso, 91100 Ii (FI); Aunio, Antti, 90410 Oulu (FI)
(74) Representative: Høiberg A/S

(56) References cited:
- WO-A1-2008/029001
- US-A1- 2011 166 631
- US-B2- 6 955 684

## Description

The present invention relates to a system and a method for changing the perception of season of an individual.

### Background of invention

It is well known that light has a direct effect on human and animal health because of the way it influences the circadian rhythm. And the human (and animal) brain includes regions that can be affected by optical radiation. This may result in a metabolic or nervous responsive stimulation, which may appear as a change in concentrations of several hormones and neuro-transmitters. The light may be natural sunlight but also artificial light sources are known to have an effect. The hormone melatonin is secreted in the pineal gland as a response to light and helps to control the circadian rhythm. Secretion of melatonin peaks at night and ebbs during the day and its presence provides information about night-length. Disruption of the circadian rhythm, e.g. due to lack of daylight or crossing time zones, usually has a negative effect and may lead to jet lag, bipolar disorders and circadian rhythm sleep disorders such as delayed sleep phase syndrome (DSPS).

It is typically necessary to use artificial optical radiation when natural light is not sufficient for achieving a desired physiological effect. Artificial optical radiation may be generated by bright light therapy devices in the form of the well-known bright light lamps and recently a more elegant solution has been commercialized in the form of the Valkee Brain Stimulation Headset described in WO 2008/029001 wherein LED (Light Emitting Diode) light is provided to the brain through the auditory canal. As disclosed in US 2010/042188 this device may be used for changing or controlling the circadian rhythm of a user.

The acute disruption in sleep quality, vigilance levels, and cognitive and athletic performance observed after transmeridian flights is presumed to be the result of a transient misalignment between the endogenous circadian pacemaker and the shifted sleep schedule. Boivin et al. (Journal of Biological Rhythms, Vol. 17 No. 3, June 2002, 266-276) demonstrated that the schedule of exposure to bright room light can substantially affect circadian adaptation to a shifted sleep-wake schedule. The subjects that were exposed to room light earlier in the day became more quickly adjusted to the destination time zone. In EP 1285676 a predetermined program controls the application of several daily doses of light applied to the body to change the circadian rhythm. A dose scheme for gradually changing the circadian rhythm of a user by means of non-ocularly applied light is disclosed in US 6,955,684.

It is generally known that bright light suppresses the output of melatonin. However, recent studies suggest that melatonin suppression can even take place down to a level of 40-80 lux. After sunset the pineal gland responds to the decreased light levels by greatly increasing its output of melatonin. After a few hours, blood melatonin levels reach a point where sleep is induced. Melatonin levels usually peak two to four hours after the onset of sleep and decrease gradually during the remaining sleep period. Daylight typically inhibits the production of melatonin, and levels of melatonin usually reach a minimum sometime during the afternoon.

The current understanding of the melatonin decrease cycle is as follows:
1. Light is observed by the eye.
2. The eye is connected via a neural pathway to the Supra Chiasmic Nucleus (SCN).
3. SCN creates an impulse
4. The impulse is transmitted via a neural (electrical / chemical) pathway to the pineal gland.
5. The pineal gland reacts to the neural impulse and stops / decreases the production of melatonin.

Thus, illumination in the form of bright light provided in the evening may cause melatonin levels to drop and the result of this melatonin decrease cycle is called melatonin suppression. Consequently melatonin suppression may have an impact on the ability to fall asleep and bright light therapy is therefore typically administered in the morning, e.g. by means of wake-up lights, or through the day. Dawn can be simulated by gradually rising the intensity of the applied light level. Likewise dusk can be simulated by gradually lowering the intensity of the applied light level, however great care has to be taken to avoid melatonin suppression, if the purpose of simulating dusk is relaxation.

### Summary of invention

Entrainment of the circadian rhythm is known to help in treatment of sleep rhythm related disorders such as jetlag, sleep irregularity caused by shift work, exceptional change of sleep rhythm and SAD. It is also known to be effective in alleviation of stress symptoms, nervous disorders caused by decreased light sensitivity of brain, improvement of plasticity of nerve system and treatment of sexual insufficiency. However, many mood disorders and neurological disorders have a pattern of symptomality changing in accordance with the annual rhythm, i.e. changing with the season. One object of the invention is therefore to be able to change the annual rhythm of a subject. This may be achieved by a system for adjusting the seasonal rhythm of an individual comprising one or more radiation units adapted to direct optical radiation at one or more neuroanatomical brain structures of the individual from at least one extra-cranial non-ocular position, and planning means for calculating a dose scheme adapted to be applied to the individual over a plurality of days, said dose scheme comprising at least a morning dose of optical radiation and at least an evening dose of optical radiation, said dose scheme adapted to gradually adjust the length of the day perceived by the individual by incorporating gradual adjustments of the time difference between morning and evening doses.

The invention can be used in a method for adjusting the seasonal rhythm of an individual comprising the steps of calculating a dose scheme adapted to be applied to the individual over a plurality of days, said dose scheme comprising at least a morning dose of optical radiation and an evening dose of optical radiation directed at one or more neuroanatomical brain structures of the individual from at least one extra-cranial non-ocular position, said dose scheme adapted to gradually adjust the length of the day perceived by the individual by incorporating gradual adjustments of the time difference between morning and evening doses, and applying said dose scheme to said individual over said plurality of days.

The present invention is based on the surprising finding that bright light directed at the brain of an individual from at least one extra-cranial non-ocular position does not cause melatonin suppression. The level of melatonin before and after light exposure can be measured in the urine or in saliva. This has been verified experimentally as will be demonstrated below. This observation is exactly opposite to the normal melatonin suppression usually seen when individuals are exposed to bright light in the evening hours. The consequence is that bright light therapy can be provided in the evening while avoiding the melatonin suppression that affects the sleep pattern of individuals. And furthermore bright light therapy can be provided during the night, e.g. via the auditory canal, without blocking melatonin secretion. It is generally known that melatonin suppression may have a negative health effect, as e.g. known from shift working. By providing the light therapy trans-cranially melatonin suppression is avoided and consequently these negative health effects can be avoided.

The knowledge of the season of the year is linked to the length of the day for many animals. I.e. if the length of the day is changed the animal's perception of the season is changed. With the present invention the gradual adjustment of the length of the day perceived by the individual shifts said individual's perception of the season. A human being living in a modern society knows the season by the calendar, which is used to organize human life. However, any human being and any animal is a biological organism influenced by photoperiodism, which is the physiological reaction of organisms to the length of day or night. Thus, with the present invention a human being's or animal's biological perception of the season can be adjusted. As many mood disorders and neurological disorders have a seasonal pattern, the adjustment of the seasonal rhythm of an individual may have an effect on these disorders.

Pronounced and persistent seasonal patterns in fertility are observed in virtually all human populations and many animals also have pronounced seasonal fertility variation. For example the southern United States show a pronounced seasonal pattern, where births decline substantially in April and May, whereas in northern Europe births increase substantially in March and April. Seasonal variations in fertility were more pronounced in earlier agricultural populations, but seasonality has increased in this century in some high income, low fertility populations such as Sweden. Summer heat may play an important role in explaining the July-August trough in conceptions in the southern United States, but e.g. temperature does not seem to play a role in explaining the pronounced June-July peak in conceptions in Sweden. Temperature also appears to be relatively unimportant in several other populations with substantial seasonal variations in births, suggesting that other factors play an important role in birth seasonality. The system according to the present invention may adjust the season of an individual, and as a consequence the fertility of an individual may be influenced by the present invention. The system according to the present invention may therefore be applied for use in increasing or decreasing the fecundity / fertility of an individual.

### Description of Drawings

The invention will now be described in further detail with reference to the drawings in which
- Fig. 1a: shows a world map with the illumination pattern in January,
- Fig. 1b: shows a world map with the illumination pattern in June,
- Fig. 1c: shows the annual difference between the length of the day at different latitudes,
- Fig. 2: shows examples of dose schemes,
- Fig. 3: shows an embodiment of the system,
- Fig. 4: show measured level of melatonin in saliva samples from test persons
- Fig. 5: shows the radiation pattern of the atmosphere, and
- Fig. 6: shows a device for providing light and heat therapy.

### Detailed description of the invention

In one embodiment of the invention the seasonal rhythm of the individual is adjusted from a present seasonal and/or daylight rhythm to a predefined seasonal and/or daylight rhythm. I.e. the seasonal rhythm of the individual.

The individual may be an animal, such as a vertebrate, such as a mammal, such as a human or a non-human animal. I.e. the invention may be applied to humans. However, the present invention can also advantageously be applied to non-human animals, e.g. livestock animals like cows, pigs, goats and chickens. As described herein the present invention may be applied for increasing the fecundity of the animals. However, also the growth or the milk output may be increased by changing and optimizing the seasonal rhythm by means of the present invention. E.g. the further north or south from equator the greater is the difference in milk output from cows between summer and winter.

The one or more radiation units may be part the device described in WO 2008/029001 and commercialized in the form of the Valkee Brain Stimulation Headset. The planning means may be incorporated or integrated in such a device or may integrated as executable software in an external device, such as a personal computer, cell phone, smartphone etc.

In the preferred embodiment of the invention a dose of optical radiation is defined by one or more of the following parameters: duration, intensity, illuminance, total power, spectral output and spectral composition.

In the preferred embodiment of the invention the dose scheme is a daily dose scheme, i.e. the dose scheme is calculated to be administered daily and typically the predefined adjustment is a gradual or regular, preferably daily, adjustment of the time difference between morning and evening doses. The calculated dose scheme is therefore preferably adapted to be applied to said individual each day of said plurality of days.

In one embodiment of the invention the morning dose differs from the evening dose by one or more of the following parameters: duration, intensity, illuminance, total power, spectral output and spectral composition. E.g. the morning dose may have more or less lux and be shorter or longer than the evening dose.

In one embodiment of the invention the dose scheme further comprises at least one noon dose adapted to be applied in between a morning dose and an evening dose. The noon dose may differ from the morning and/or evening dose by one or more of the following parameters: duration, intensity, illuminance, total power, spectral output and spectral composition.

In one embodiment of the invention the system is adapted to be applied to the individual when said individual is awake and/or when said individual is sleeping. Both options are possible because the optical radiation is applied non-ocularly, i.e. the eyes of the individual can be open or closed.

In a further embodiment of the invention the dose scheme further comprises at least one night dose adapted to be applied in between an evening dose and a morning dose. The night dose may differ from the morning and/or evening and/or noon dose by one or more of the following parameters: duration, intensity, illuminance, total power, spectral output and spectral composition.

In one embodiment of the invention the dose scheme is adapted to a predefined geographical location, such as the geographical location of the individual. The length of the day through a year varies with the season but it is also depending on the latitude. I.e. the further north or south of equator, the seasonal variation in day length increases. Thus, to emulate spring season the perceived daytime of the user is extended each day by incremental increase of the time difference between morning and evening doses. As long as the derivative of this time difference is positive the brain, CNS and body will interpret it as spring (and vice versa for fall). To prolong the feeling of spring only small day to day incremental steps can therefore be provided. This can be important for e.g. livestock breeding where the fertility may be highest during springtime.

In one embodiment of the invention an alarm is provided for attaining the attention of the individual when a scheduled dose of optical radiation is to be applied.

In one embodiment of the invention the optical radiation is in the form of bright light, such as visible light with wavelengths in the visible spectrum. The optical radiation may also be in the form of infrared light. In a further embodiment the optical radiation is in the form of both visible light and infrared light.

As described in further detail below the present inventors have observed the surprising effect that bright light provided non-ocularly, e.g. through the ear-canal, does not result in melatonin suppression. This is in contrast to the melatonin suppression observed when bright light is provided through the eyes. Test persons have also been observed to fall asleep easier when heat in the form of infrared radiation is provided through the auditory canal, which might indicate an increase in the level of melatonin. It therefore seems that bright light and/or heat provided non-ocularly does not cause melatonin suppression and in some cases even cause an increase in the level of melatonin.

In a further embodiment of the invention one or more sources of heat is therefore provided. The source of heat, such as an infrared light source or a resistor, is preferably adapted to transfer heat to said one or more neuroanatomical brain structures of the individual from said at least one extra-cranial non-ocular position.

A further aspect of the invention therefore relates to a device for non-invasive sleep therapy comprising one or more radiation units adapted to direct infrared radiation through the auditory canal of a user, said device applied for use in inducing sleep of said user.

Another embodiment of the invention relates to a device for non-invasive sleep therapy comprising one or more radiation units adapted to direct infrared radiation through the auditory canal of a user, said device applied for use in increasing the concentration of melatonin in the body of the user.

In one embodiment of the invention a dose of light is light provided with an intensity of between 3 and 9 lumens, such as 3, 4, 5, 6, 7, 8 and 9 minutes, for 6 to 12 minutes, i.e. for 6, 7, 8, 9, 10, 11 or 12 minutes. E.g. using a device as disclosed in WO 2008/029001.

Another embodiment of the invention therefore relates to a device for non-invasive sleep therapy comprising one or more sources of heat adapted to direct thermal radiation through the auditory canal of a user or adapted to transfer heat to the auditory canal of the user, said device applied for use in inducing sleep of said user.

Another embodiment of the invention therefore relates to a device for non-invasive sleep therapy comprising one or more sources of heat adapted to direct thermal radiation through the auditory canal of a user or adapted to transfer heat to the auditory canal of the user, said device applied for use in increasing the concentration of melatonin in the body of the user.

In one embodiment of the invention said one or more radiation units comprise a source of bright light with wavelengths in the visible spectrum and a source of infrared light suitable for providing thermal radiation / heat radiation.

In one embodiment of the invention said one or more radiation units comprise a source of bright light and a source of heat, such as a heat source in the form of a resistor.

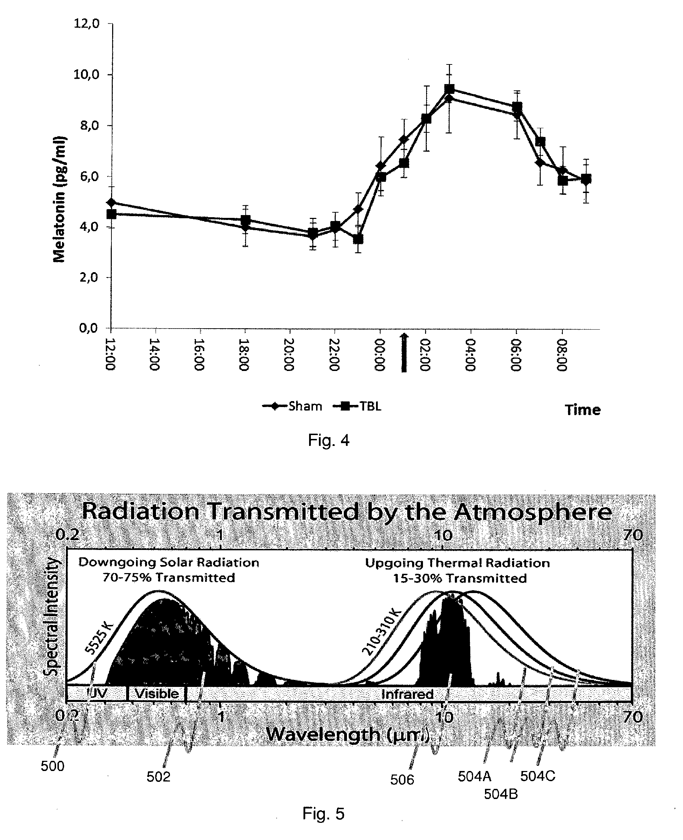

As a consequence of the surprising abovementioned observation of avoiding melatonin: In a further embodiment of the invention the evening dose and/or the night dose comprise a greater absolute and/or relative amount of infrared light compared to the morning dose and/or the noon dose. Thus, the evening dose may comprise the greatest amount of infrared light. Alternatively only the evening dose and/or the night dose comprise infrared light. The infrared light is preferably adapted to transfer heat to said individual. Thus, preferably the amount of "visible radiation area" 500 or 502 (of figure 5) relative to the "heat radiation area 506 or 504A, 504B, 504C can be adjusted.

The infrared light may be mid-wave infrared or long-wave infrared. Thus, the wavelength of the infrared light may be at least 3 µm, or at least 5 µm, or at least 6 µm, or at least 7 µm, or at least 8 µm, or at least 9 µm, or at least 10 µm, or at least 12 µm, or at least 15 µm, or at least 20 µm, or at least 30 µm, or at least 40 µm, or at least 50 µm, or at least 100 µm.

Furthermore, the optical radiation may cover a spectrum from the low ultraviolet (UV) area of about 0.2 µm to the infrared area of about 70 µm.

Furthermore, the evening and/or night doses might have more heat compared to morning and/or noon doses. The increased amount of heat may originate from thermal radiation by having an increased amount of infrared light in the optical radiation. This may be provided by having a dedicated infrared light source or by changing the spectrum of a light source of by filtering a light source comprising both visible light and infrared light. The heat may also originate from a heating element working on the principle of Joule heating, i.e. an electric current through a resistor converting electrical energy directly into heat energy.

In one embodiment of the invention control means is provided for controlling the intensity, illuminance and/or spectral composition of the optical radiation. A housing in optical and/or electrical connection with said one or more radiation units and one or more light sources for generating the optical radiation may furthermore be provided.

In one embodiment of the invention the optical radiation emitted from a radiation unit is applied with a predefined spectral composition. Furthermore, the optical radiation emitted from a radiation unit may be applied with one or more of the following parameters being predefined for a plurality of predefined wavelength intervals: duration, intensity, total power, spectral output and spectral composition.

In one embodiment of the invention the optical radiation emitted from a radiation unit is applied with a predefined ratio of:
a first spectral output integrated over a first wavelength interval, and
a second spectral output integrated over a second wavelength interval.

In one embodiment of the invention the extra-cranial position is below the cerebrum of the individual.

In one embodiment of the invention at least one radiation unit is adapted to be arranged such that at least part of said optical radiation is guided trans-cranially through the at least one of the followings cranial bones: the temporal bone, squama temporalis of the temporal bone, mastoid portion of the temporal bone, petrous portion of the temporal bone, tympanic part of the temporal bone, the zygomatic bone, the sphenoid bone, the frontal bone, the parietal bone.

In one embodiment of the invention said one or more neuro-anatomical brain structures are selected from the group of: suprachiasmatic nucleus (SCN), globus pallidus, striatum, the brain stem, medulla oblongata, pons, midbrain, raphe nuclei, nucleus raphe obscurus, raphe magnus, raphe pontis, raphe pallidus, nucleus centralis superior, nucleus raphe dorsalis, nuclei linearis intermedius and linearis rostralis, frontal cortex, temporal lobes, cingular cortex, parietal lobe, postcentral area and prae, occipital lobe, hippovampus, hypothalamus, striatum, thalamus, mesencephalon including substantia nigra, pons incl. raphe nucleus, medulla, cerebellum (vermis), cerebellar cortex, medulla spinalis, pituitary gland and pineal gland.

### Examples

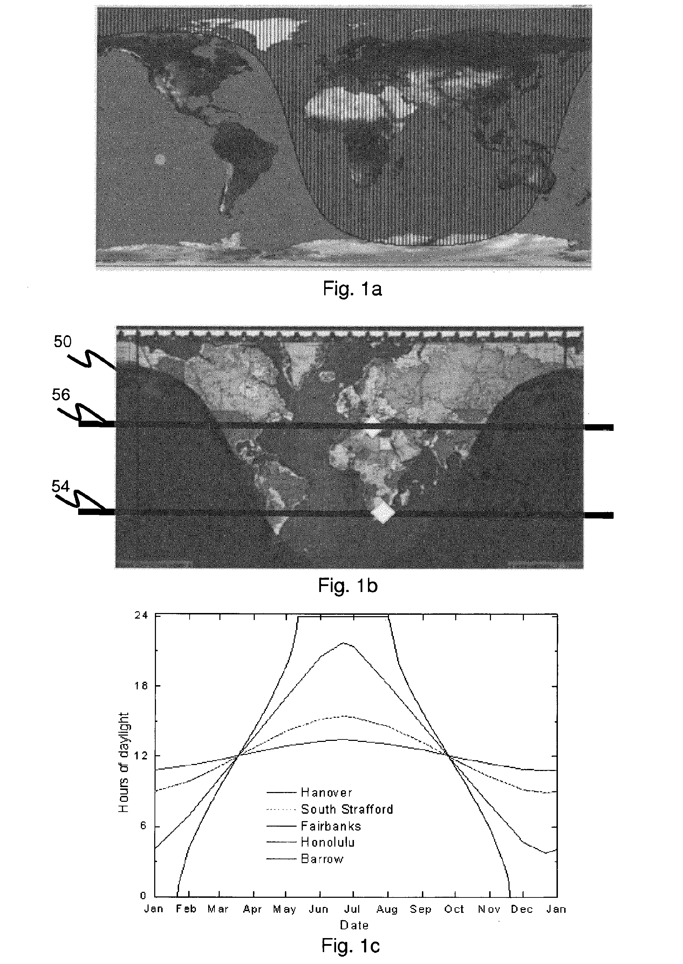

The illumination level ratio between nighttime and daytime varies depending on the season and the latitude. This is illustrated in fig. 1 with the worldwide sunlight illumination in January at time of noon in California shown in fig. 1 a and worldwide sunlight illumination in June at time of noon in London shown in fig. 1 b. Fig. 1c shows the length of the day during the year in different cities. In Honolulu close to equator the annual variation is small whereas Barrow in Alaska having a large variation with total darkness January and 24 hours of daylight in July.

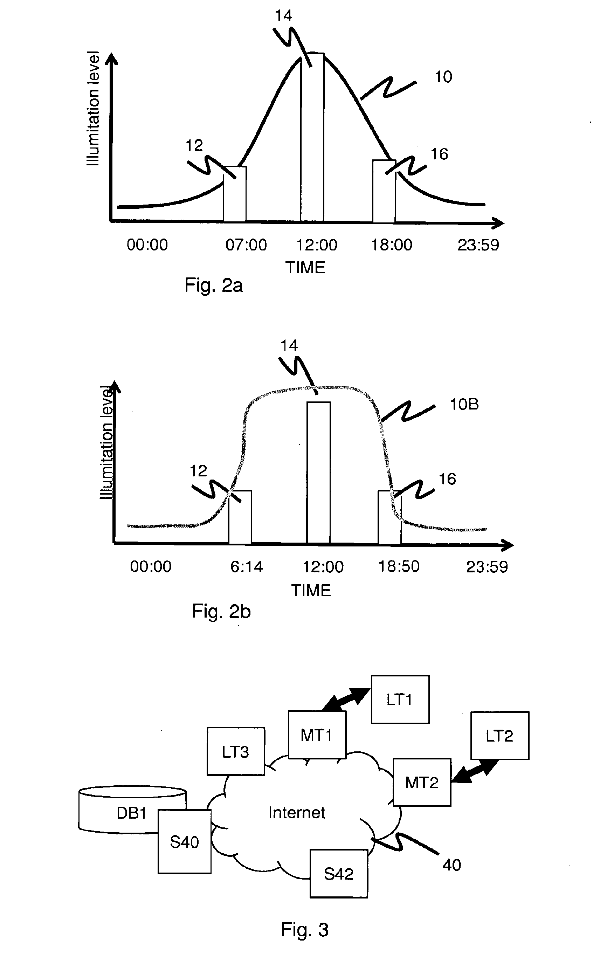

Figure 2a shows an example of a dose scheme adjusted to a specific outdoor daylight pattern illustrated as the curve 10. During night the illumination level is low, because there is no daylight. During the day the illumination level is higher with peak illumination at noon. This daily dose scheme comprises a morning dose 12 (e.g. 15 minutes between 7 and 7:15), a noon dose 14 and an evening dose 16. A dose refers to amount of light (energy) given to user and the noon dose 14 is seen to have a higher illumination to emulate the peak in the daylight pattern 10.

The outdoor daylight pattern 10 may be the daylight pattern of a specific travel destination for a person. This person may currently be in a different time zone on a different latitude and by applying the system according to the invention he may gradually adjust to the daylight pattern of the destination before he travels. Compared to the prior art in this field, the present invention can adjust the circadian and seasonal rhythm of the person to the destination daylight pattern in a much more accurate way. Prior art devices of jet lag adjustment typically provides a morning dose which can be gradually adjusted to the morning time of the destination, but with the present invention several daily doses can be provided to simulate the destination daylight pattern, in particular an evening dose can be provided without inducing melatonin suppression.

A further embodiment of the system according to the invention may comprise a user interface for selecting the time of the year (i.e. the season) and place of the earth, e.g. a travel destination. The latitude of the place and the selected time of the year provide the length of the day and typical illumination levels during the day in the target season and place. This derived illumination level is used as input for calculating a dose scheme to be applied. For example person living in Finland in November would like to feel like he is in Honolulu in April. The system would use latitude information to calculate sunrise and sunset times in the target (for example in April 14 2011 the sun rises at 6:14AM and sets at 6:50PM, a Honolulu April day illustrated in fig. 2b with the daylight curve 10b). An input to the system will typically also include the number of days to apply the dose scheme. This number of days may be provided automatically by the system or may be a user input. The planning means then calculates a dose scheme which when applied over a number of days gradually transforms the user's circadian and seasonal rhythm from Finland in November to Honolulu in April.

E.g. if the goal is to simulate May in Rome. The sunrise May 1^{st} is 5:07AM and sunset was 19:08 in Rome in 2011. The individual is located in Helsinki, Finland and the time of the year is December 2011, i.e. with sunrise 9:05 AM and sunset at 15:16 PM.
As dose scheme with morning and evening light doses could then be:
Day 1: 9:00 and 15:30
Day 2: 8:00 and 16:30
Day 3: 7:00 and 17:30
Day 4: 6:00 and 18:30
Day 5: 5:00 and 19:30
for the individual in Helsinki in December wanting to feel like in Rome in spring (May), i.e. the transfer to "Rome cycle" is provided in five days. The morning dose can be adjusted to "normal" waking up times of the person, i.e. the morning dose could be given later if the individual is still sleeping.

A further embodiment of the invention is shown in fig. 3 where systems LT1, LT2, LT3 according to the present invention are connected to a server S40 (for example a web service / server such as Apache etc. based server) with database DB1 connected to the internet 40. The systems LT1, LT2, LT3 can be connected to a service running in the server S40 via the internet 40 either directly (LT3) using integrated communication means (such as WLAN, wireless modem (WCDMA, GSM etc.)) or via PC/laptop/smartphone or similar (MT1, MT2). There can be 3^{rd} party service running in server system S42 such as a Facebook™ service.

When using the service users of the systems LT1, LT2, LT3 can be connected via the internet directly to the server S40 or via Application Program Interface (API) via S42. One of the services running in the server S40 could be a "jet-lag" application. The application can have a user interface (UI) for a user to enter destination and departure places. The UI is preferably based on a map interface allowing users to enter destination in user friendly way. Departure (i.e. current location) can be entered via UI as well or it can be derived automatically using geo location means such as GPS, reverse IP mapping etc. The server S40 calculates sunrise and sunset times of the destination and these values are used to calculate a dose scheme.

Another service running in the server S40 could be a "season selector" application. The user of the device selects a desired season, for example using map interface 50 as illustrated in fig. 1 b. For example summer time in Rome (marked in fig. 1b with diamond along longitude line 56). The server S40 calculates illumination patterns in Rome for said season and a dose scheme can be calculated.

In a placebo-controlled study of the relation between trans-cranial bright light (TBL) and melatonin suppression, eight subjects (age: 27 ± 5 years) with normal circadian rhythm participated. During two study sessions saliva samples were collected hourly between 9pm-3am and 6am-9am, at noon and 6pm. The subjects were exposed to 24 minutes of trans-cranial bright light (using the Valkee brain stimulation headset) or sham placebo exposure via the auditory canals at 1.10 am. The condition order was randomized. The concentration of melatonin in the saliva samples was measured using radioimmunoassay. As presented in fig. 4 the results of this study shows that there were no significant differences in mean saliva (p=0.435) concentration between sessions in any sampling points. As seen in fig. 4 the secretion of melatonin during trans-cranial bright light (TBL) and sham sessions paralleled natural secretion profile of human. It is therefore concluded that trans-cranially administered bright light does not suppress melatonin secretion.

One explanation for such surprising effect might be related to heat. Figure 5 shows radiation transmitted by the atmosphere. The sun emits radiation toward the earth as shown with line 500 (5525 Kelvin radiation). Part of the light is absorbed or scattered in the atmosphere. The part that reaches the earth surface is indicated with area 502. As one can see most of the spectral intensity is in "visible spectrum" area. The radiation 502 that reaches the earth surface is absorbed by earth, buildings, water, rocks etc. and transformed to heat. The heat radiation from the earth is marked with 504A, 504B and 504C. Part of the "outgoing" radiation from the earth is further absorbed on the atmosphere as indicated with area 506. This radiation 506 has main peak wavelength around 10 µm.

The amount of radiation 506 is less in the morning since the earth cools down during the night. The amount of radiation 506 increases during the day and reaches a maximum before sunset. Due to heat capacity of the earth the radiation 506 continues also after the sun set. The total intensity of 506 goes down towards morning.

Based on experiments the radiation 506 type of illumination towards patients has been beneficial when providing treatments relating to jetlag, seasonal disorders or when adjusting circadian clock or when adjusting seasonal clock. Thus, in a further embodiment of the invention bright light therapy is combined with heat radiation resembling pattern 506.

An experiment was conducted with a female subject (38 years old). She had difficulties to fall asleep after waking up in middle of the night (caused by baby crying). In a set of tests the subject was provided with infrared optical radiation via the auditory canal with a wavelength of around 10 µm, i.e. in practice heat radiation. Subject was observed to fall asleep significantly easier.

According to a further embodiment of the invention light source might include mid infrared i.e. heat (range around 10 µm) to simulate evening after the sunset. Based on experiments it has been observed that these wavelengths causes subject to fall asleep easier thus also helping with sleep disorders. In further embodiment of the device LED can be accompanied and/or replaced with a heat source, e.g. a resistor, in order to generate heat, or another light source having a poor efficiency in the visible spectrum. The amount of heat generated by the device can be controlled by e.g. pulsing the light source (providing for example pulse width modulation PWM) and/or the heat source, and controlling for example duration, intensity, pulse frequency, total power, current, etc. applied to the light source or the heat source.

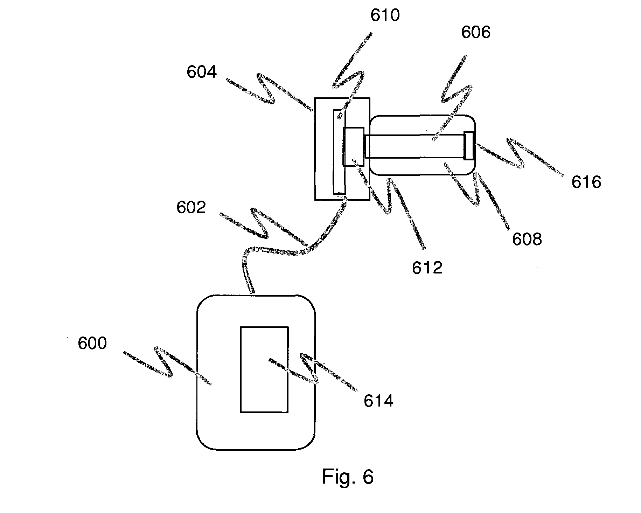

An example device for providing combination of light and heat treatment is shown in Figure 6. The device can consist of control unit 600. The control unit 600 can contain a battery for providing power, external connectors and interfaces (both wired and wireless) for charging the batteries and configuring the device, programmable logic or microprocessor, a user interface 614 for controlling and configuring the device. Control unit 600 provides electricity for via wire 602 to at least one ear plug unit 604 (preferably two i.e. one for left and other for right ear). The ear plug unit 604 consists of printed circuit board (PCB) 610. Light emitting diodes (LED)'s can be mounted in said PCB for providing light for bright light therapy. The light is guided to ear via light guide 606. There can be soft material such as silicon 608 for ensuring proper fit of the device into the ear canal. The soft material 608 may be adapted such that heat generated by LED is transferred to the auditory ear canal in order to provide heat in addition to visible light to the brain.

Furthermore, there may be one or more resistors mounted in the PCB 610 as a dedicated heat source for providing heat to the auditory canal. In one embodiment of the invention the device has both LED and resistor, where the LED with visible light is used mainly during morning and the resistor for generating heat is used mainly during the evening and/or night time.

In further embodiment of the invention there can be a liquid crystal display (LCD) type of element 616 embedded or installed at the end of the light guide 606. The LCD 616 can be controlled to allow radiation emitted from an LED (or other light source) to go through or to be absorbed in the element. If the LCD is turned on OFF stage it blocks the light thus transforming it to heat like a resistor. This structure enables a control of the amount of heat versus the amount of visible light to be provided to the brain when using said device.

Said device which combines visible light and heat both provided to patient / person non-ocularily can be used, in addition to traditional light therapy of "waking up a person", to help persons/patients to fall asleep thus reducing sleep disorders.

## Claims

1. A system for adjusting the seasonal rhythm of an individual comprising
- one or more radiation units adapted to direct optical radiation at one or more neuroanatomical brain structures of the individual from at least one extra-cranial non-ocular position, and
- planning means for calculating a dose scheme,
wherein
the dose scheme is adapted to be applied to the individual over a plurality of days, the system being charaterized in that
said dose scheme comprises at least a morning dose and at least an evening dose of optical radiation adapted to be delivered to said one or more neuroanatomical brain structures of the individual through said one or more radiation units, and
said dose scheme is adapted to gradually adjust the length of the day perceived by the individual by incorporating predefined adjustments of the time difference between morning and evening doses.

2. The system according to claim 1, wherein the seasonal rhythm of the individual is adjusted from a present seasonal and/or daylight rhythm to a predefined seasonal and/or daylight rhythm.

3. The system according to any of preceding claims, wherein the dose scheme is a daily dose scheme and wherein the dose scheme is adapted to be applied to said individual each day of said plurality of days.

4. The system according to any of preceding claims, wherein the dose scheme is adapted to a predefined geographical location, such as the geographical location of the individual.

5. The system according to any of preceding claims, wherein said one or more radiation units is adapted such that the optical radiation is in the form of visible bright light, infrared light or a combination of visible bright light and infrared light.

6. The system according to claim 5, wherein the infrared light is adapted to transfer heat to said individual.

7. The system according to any of claims 5 to 6, wherein said one or more radiation units are adapted such that the amount of visible bright light relative to the amount of infrared light can be adjusted.

8. The system according to any of preceding claims, wherein said one or more radiation units comprise a source of bright visible light and a source of infrared light.

9. The system according to any of preceding claims, wherein said one or more radiation units comprise a source of bright visible light and a source of heat, such as a resistor.

10. The system according to any of preceding claims, wherein the evening dose comprises a greater absolute and/or relative amount of infrared light and/or heat compared to the morning dose.

11. The system according to any of claims 5 to 10, wherein the wavelength of the infrared light is at least 7 µm.

12. The system according to any of claims 10 to 11, wherein the evening dose is applied for use in increasing the concentration of melatonin in the body of the user.

13. The system according to any of preceding claims, wherein the extra-cranial position is the auditory canal.

14. The system according to any of preceding claims, for use in increasing the fecundity of the individual.

## Patentansprüche

1. System zur Einstellung des Jahreszeiten-Rhythmus eines Individuums, welches umfasst:
- ein oder mehrere Strahlungseinheiten, die angepasst sind, optische Strahlung zu einem oder mehreren neuroanatomischen Gehirnstrukturen eines Individuums von mindestens einer extrakraniellen, nicht-okularen Position zu leiten, und
- Planungsmittel zur Berechnung eines Dosis-Schemas, worin das Dosis-Schema angepasst ist, einem Individuum über mehrere Tage verabreicht zu werden,
worin das System **dadurch gekennzeichnet ist, dass**
das Dosis-Schema mindestens eine Morgen-Dosis und mindestens eine Abend-Dosis optischer Strahlung umfasst, die angepasst ist, zu der einen oder den mehreren neuroanatomischen Gehirnstrukturen des Individuums über die eine oder mehrere Strahlungseinheiten geliefert zu werden, und
worin das Dosis-Schema angepasst ist, die Länge des durch das Individuum erfassten Tages allmählich einzustellen, indem bestimmte Einstellungen des Zeitunterschieds zwischen den Morgen- und Abend-Dosen verändert werden.

2. System nach Anspruch 1, worin der Jahreszeiten-Rhythmus des Individuums von einem bestehenden Jahreszeiten- und/oder Tageslicht-Rhythmus zu einem bestimmten Jahreszeiten- und/oder Tageslicht-Rhythmus eingestellt wird.

3. System nach einem der vorstehenden Ansprüche, worin das Dosis-Schema ein TagesDosis-Schema ist und worin das Dosis-Schema angepasst ist dem Individuum jeden Tag der mehreren Tage verabreicht zu werden.

4. System nach einem der vorstehenden Ansprüche, worin das Dosis-Schema an eine bestimmte geographische Örtlichkeit angepasst ist, wie die geographische Örtlichkeit des Individuums.

5. System nach einem der vorstehenden Ansprüche, worin die eine oder mehrere Strahlungseinheiten angepasst sind, so dass die optische Strahlung in Form von sichtbarem hellem Licht, Infrarot oder einer Kombination von sichtbarem hellem Licht und Infrarot ist.

6. System nach einem Anspruch 5, worin das Infrarot angepasst ist dem Individuum Wärme zuzuführen.

7. System nach einem der Ansprüche 5 bis 6, worin die eine oder mehrere Strahlungseinheiten angepasst sind, dass die Menge an sichtbarem hellem Licht relativ zur Menge an Infrarot eingestellt werden kann.

8. System nach einem der vorstehenden Ansprüche, worin die eine oder mehrere Strahlungseinheiten eine Quelle sichtbaren hellen Lichts und eine Quelle für Infrarot umfassen.

9. System nach einem der vorstehenden Ansprüche, worin die eine oder mehrere Strahlungseinheiten eine Quelle sichtbaren hellen Lichts und eine Wärmequelle, wie einen Widerstand, umfassen.

10. System nach einem der vorstehenden Ansprüche, worin die Abend-Dosis eine größere absolute und/oder relative Menge Infrarot und/oder Wärme im Vergleich zur Morgen-Dosis umfasst.

11. System nach einem der Ansprüche 5 bis 10, worin die Wellenlänge des Infrarots mindestens etwa 7 µm beträgt.

12. System nach einem der Ansprüche 10 bis 11, worin die Abend-Dosis zur Erhöhung der Konzentration an Melatonin in dem Körper des Verwenders eingesetzt wird.

13. System nach einem der vorstehenden Ansprüche, worin die extrakranielle Position der Hörkanal ist.

14. System nach einem der vorstehenden Ansprüche zur Verwendung bei der Erhöhung der Fertilität des Individuums.

## Revendications

1. Système pour ajuster le rythme saisonnier d'un individu comprenant :
une ou plusieurs unités de rayonnement adaptées pour diriger le rayonnement optique au niveau d'une ou de plusieurs structures cérébrales neuroanatomiques de l'individu à partir d'au moins une position non oculaire extra-crânienne, et
des moyens de planification pour calculer un programme de dosage,
dans lequel :
le programme de dosage est adapté pour être appliqué sur l'individu sur une pluralité de jours, le système étant **caractérisé en ce que** :
ledit programme de dosage comprend au moins une dose le matin et au moins une dose le soir de rayonnement optique adapté pour être délivré auxdites une ou plusieurs structures cérébrales neuroanatomiques de l'individu par lesdites une ou plusieurs unités de rayonnement, et
ledit programme de dosage est adapté pour ajuster progressivement la longueur du jour perçu par l'individu en incorporant des ajustements prédéfinis de différence de durée entre les doses du matin et du soir.

2. Système selon la revendication 1, dans lequel le rythme saisonnier de l'individu est ajusté à partir d'un rythme saisonnier et/ou de lumière du jour actuel jusqu'à un rythme saisonnier et/ou de lumière du jour prédéfini.

3. Système selon l'une quelconque des revendications précédentes, dans lequel le programme de dosage est un programme de dosage quotidien et dans lequel le programme de dosage est adapté pour être appliqué sur ledit individu chaque jour de ladite pluralité de jours.

4. Système selon l'une quelconque des revendications précédentes, dans lequel le programme de dosage est adapté à un emplacement géographique prédéfini, tel que l'emplacement géographique de l'individu.

5. Système selon l'une quelconque des revendications précédentes, dans lequel lesdites une ou plusieurs unités de rayonnement sont adaptées de sorte que le rayonnement optique se présente sous la forme d'une lumière vive visible, une lumière infrarouge ou une combinaison de lumière vive visible et d'une lumière infrarouge.

6. Système selon la revendication 5, dans lequel la lumière infrarouge est adaptée pour transférer la chaleur audit individu.

7. Système selon l'une quelconque des revendications 5 à 6, dans lequel lesdites une ou plusieurs unités de rayonnement sont adaptées de sorte que la quantité de lumière vive visible par rapport à la quantité de lumière infrarouge peut être ajustée.

8. Système selon l'une quelconque des revendications précédentes, dans lequel lesdites une ou plusieurs unités de rayonnement comprennent une source de lumière visible vive et une source de lumière infrarouge.

9. Système selon l'une quelconque des revendications précédentes, dans lequel lesdites une ou plusieurs unités de rayonnement comprennent une source de lumière visible vive et une source de chaleur, telle qu'une résistance.

10. Système selon l'une quelconque des revendications précédentes, dans lequel la dose du soir comprend une quantité absolue et/ou relative supérieure de lumière infrarouge et/ou de chaleur par rapport à la dose du matin.

11. Système selon l'une quelconque des revendications 5 à 10, dans lequel la longueur d'onde de la lumière infrarouge est d'au moins 7 µm.

12. Système selon l'une quelconque des revendications 10 à 11, dans lequel la dose du soir est appliquée pour être utilisée afin d'augmenter la concentration de mélatonine dans le corps de l'utilisateur.

13. Système selon l'une quelconque des revendications précédentes, dans lequel la position extra-crânienne est le canal auditif.

14. Système selon l'une quelconque des revendications précédentes, destiné à être utilisé pour augmenter la fécondité de l'individu.
